# EUROPEAN PATENT SPECIFICATION

(11) **EP 3 435 975 B1**
(45) Date of publication and mention of the grant of the patent: **15.09.2021**
(21) Application number: 17716352.4
(22) Date of filing: 27.03.2017
(51) Int. Cl.: A61K 9/06, A61K 47/36

(54) **INJECTABLE COMPOSITIONS AND METHODS OF PREPARATION AND USE THEREOF**
INJIZIERBARE ZUSAMMENSETUNG SOWIE DEREN HERSTELLUNGSMETHODE UND VERWENDUNG
COMPOSITION INJECTABLE ET PROCÈS DE PRÉPARATION ET SON UTILISATION

(30) Priority: 01.04.2016 US 201662316891 P
(43) Date of publication of application: 06.02.2019
(73) Proprietor: Boston Scientific Scimed, Inc., Maple Grove, MN 55311 (US)
(72) Inventor: RAYBIN, Samuel, Marlborough, MA 01752 (US); HOLLYER, Matthew, B., Williamstown, VT 05679 (US)
(74) Representative: Vossius & Partner Patentanwälte Rechtsanwälte mbB
(86) International application number: PCT/US2017/024259
(87) International publication number: WO 2017/172588

(56) References cited:
- EP-A1- 2 783 709
- WO-A1-99/15211
- US-A1- 2012 301 436
- US-A1- 2015 010 603
- US-A1- 2015 099 928
- HAEYEON LEE ET AL: "Optimizing gelling parameters of gellan gum for fibrocartilage tissue engineering", JOURNAL OF BIOMEDICAL MATERIALS RESEARCH. PART B: APPLIED BIOMATERIALS, vol. 98B, no. 2, 20 August 2011 (2011-08-20), pages 238-245, XP055385990, US ISSN: 1552-4973, DOI: 10.1002/jbm.b.31845
- JO?O T. OLIVEIRA ET AL: "Gellan Gum Injectable Hydrogels for Cartilage Tissue Engineering Applications: In Vitro Studies and Preliminary In Vivo Evaluation", TISSUE ENGINEERING PART A, vol. 16, no. 1, 1 January 2010 (2010-01-01), pages 343-353, XP055385996, US ISSN: 1937-3341, DOI: 10.1089/ten.tea.2009.0117
- PACELLI SETTIMIO ET AL: "Injectable and photocross-linkable gels based on gellan gum methacrylate: A new tool for biomedical application", INTERNATIONAL JOURNAL OF BIOLOGICAL MACROMOLECULES, ELSEVIER BV, NL, vol. 72, 27 October 2014 (2014-10-27), pages 1335-1342, XP029110626, ISSN: 0141-8130, DOI: 10.1016/J.IJBIOMAC.2014.10.046

## Description

### CROSS-REFERENCE TO RELATED APPLICATIONS

This application claims the benefit of priority to U.S. Provisional Application No. 62/316,891, filed on April 1, 2016.

### TECHNICAL FIELD

Embodiments of the present disclosure relate generally to compositions suitable for injection, methods of preparation thereof, and devices comprising such compositions.

### BACKGROUND

Various medical procedures are used for diagnosis and/or treatment of tissue. For example, an endoscopic procedure may be performed to take tissue samples from the gastrointestinal (GI) tract or other organ systems for pathological evaluation and/or therapeutic purposes, such as detection and removal of pre-cancerous mucosal tissue or tumors. Yet, removing select portions of tissue from a patient with minimal disturbance to underlying anatomy can be challenging.

In medical procedures such as endoscopic mucosal resection (EMR) and endoscopic submucosal dissection (ESD), a fluid may be injected into tissue to separate different tissue layers to assist in the removal of lesions. For example, a fluid may be injected to separate submucosal tissue from mucosal tissue. The injected fluid generally elevates the target tissue from underlying tissue layers to allow a physician to more easily resect the target tissue. Yet, fluids used for this purpose, such as saline, tend to dissipate within a few minutes, and can require periodic re-injection to ensure the target tissue remains raised throughout the procedure. More viscous injection solutions have been identified, but these alternatives are often costly, difficult to inject, and/or also prone to dissipation/breaking down too soon after injection.

As an example, EP 2 783 709 A1 describes a submucosal cushioning agent in form of an injectable Gellan gum hydrogel.

### SUMMARY OF THE DISCLOSURE

The invention for which protection is sought is defined by the independent claim(s). The dependent claims concern the particular embodiments of the invention. Disclosure that does not fall within the scope of the claims should be read as being presented for illustrative purpose.

The present disclosure includes compositions useful for tissue resection procedures and methods of preparing such compositions. According to some aspects of the present disclosure, the composition may comprise a gel formed from a polysaccharide such as gellan gum, water, and a salt as a source of monovalent or divalent cations. The gel may be allowed to set undisturbed, e.g., in a reservoir, to form a continuous, three-dimensional network prior to injection from the reservoir to a patient. The continuous, three-dimensional network may provide for a homogeneous structure of the gel.

The present disclosure includes, for example, a method of preparing a composition for delivery to a target site of a patient, wherein the method comprises combining gellan gum, at least one salt, and water to form a mixture; heating the mixture; introducing the mixture into a reservoir; and allowing the mixture to cool while inside the reservoir to form a gel having a continuous, three-dimensional structure inside the reservoir; wherein the composition is biocompatible and injectable from the reservoir through a needle to the target site. According to some aspects, the composition comprises 0.01% to 2.0% gellan gum by weight with respect to the total weight of the composition, or 0.05% to 0.5% gellan gum by weight with respect to the total weight of the composition. In some aspects, the composition may comprise one or more additional agents, such as a coloring agent.

The mixture may be introduced into the reservoir after heating the mixture at a temperature ranging from about 70°C to about 130°C. According to some aspects, the temperature of the mixture when being introduced into the reservoir may range from about 70°C to about 130°C. According to some aspects, the mixture may be allowed to cool before introducing the mixture into the reservoir. For example, after allowing the mixture to cool and introducing the mixture into the reservoir, the mixture may be heated at a temperature ranging from about 70°C to about 130°C while inside the reservoir. In some examples, heating the mixture may sterilize the mixture, such that the gel formed inside the reservoir is sterilized. For example, the mixture may be heated at or to a temperature of about 121°C while inside the reservoir.

The reservoir may be a component of a medical device or system. According to some aspects, for example, the reservoir may be a barrel of a syringe, or may comprise a flexible pouch. In some aspects, the reservoir may be coupled to a needle via a flexible tube. The gel may form a continuous, three-dimensional network across an entire cross-sectional dimension of the reservoir. For example, if the reservoir comprises a cylindrical barrel of a syringe, the continuous, three-dimensional structure of the gel may extend across an entire diameter of the reservoir. The present disclosure is not limited to cylindrical-shaped reservoirs, however, and other cross-sectional shapes are contemplated and encompassed herein.

According to some aspects, the method may be used to prepare a medical device. For example, the present disclosure includes a medical device prepared according to any of the aspects of the methods discussed herein. Such a medical device may comprise, for example, the reservoir containing the composition and a needle through which the composition may be injected. Optionally, the medical device may comprise a flexible tube connecting the reservoir to the needle. In some aspects, the medical device may comprise a syringe, such that the reservoir is provided by a barrel of the syringe, or the reservoir may be provided by a flexible pouch. The composition may be biocompatible. For example, the composition may comply with governmental regulations for pharmaceutical compositions and/or governmental regulations for medical devices. According to some aspects, the composition and/or the medical device comprising the composition may have an endotoxin level of 20 endotoxin units (EU) or less, e.g., 15 EU or less, 10 EU or less, or 5 EU or less.

In some aspects, the present disclosure includes a medical device comprising a needle; a reservoir coupled to the needle; and a composition comprising 0.01% to 2.0% gellan gum by weight with respect to a total weight of the composition; at least one salt; and water. The composition of the medical device may be prepared by combining the gellan gum, the at least one salt, and the water to form a mixture; heating the mixture; introducing the mixture into the reservoir; and allowing the mixture to cool and increase in viscosity while inside the reservoir to form a homogeneous gel; wherein the composition is injectable through the needle. The medical device may be configured such that composition is injectable from the reservoir through the needle to a target site of a patient. According to some aspects, for example, the composition may be allowed to set into a gel while inside the reservoir to form a continuous, three-dimensional network, and may not be transferred outside the reservoir prior to injection through the needle. The continuous, three-dimensional structure of the gel may extend across an entire cross-sectional dimension of the reservoir.

According to some aspects, the composition of the medical device may comprise 0.01% to 2.0% gellan gum by weight or 0.05% to 0.5% gellan gum by weight with respect to the total weight of the composition. Further, for example, the at least one salt of the composition may comprise one or more cations such as sodium, calcium, and/or magnesium cations. In some examples, the composition may comprise at least one sodium salt, at least one calcium salt, at least one magnesium salt, or a combination thereof. Additional salts providing for biocompatible compositions are also contemplated. The composition may additionally or alternatively comprise at least one coloring agent. In some examples, the medical device may comprise a syringe, e.g., the gel forming a continuous, three-dimensional network across an entire cross-sectional dimension of the barrel of the syringe. In some examples, the reservoir of the medical device may be coupled to the needle via a flexible tube, or the reservoir may be directly attached to the needle.

### BRIEF DESCRIPTION OF THE FIGURES

The accompanying drawings, which are incorporated in and constitute a part of this specification, illustrate various exemplary aspects of the disclosure, and together with the description serve to explain the principles of the present disclosure.
Figs. 1A-1C show exemplary medical devices in accordance with certain aspects of the present disclosure.
Figs. 2A-2E illustrate an exemplary tissue resection procedure in accordance with certain aspects of the present disclosure.

### DETAILED DESCRIPTION

Particular aspects of the present disclosure are described in greater detail below. The terms and definitions provided herein control, if in conflict with terms and/or definitions incorporated by reference.

As used herein, the terms "comprises," "comprising," or any other variation thereof are intended to cover a non-exclusive inclusion, such that a process, method, composition, article, or apparatus that comprises a list of elements does not include only those elements, but may include other elements not expressly listed or inherent to such process, method, composition, article, or apparatus. The term "exemplary" is used in the sense of "example" rather than "ideal."

As used herein, the singular forms "a," "an," and "the" include plural reference unless the context dictates otherwise. The terms "approximately" and "about" refer to being nearly the same as a referenced number or value. As used herein, the terms "approximately" and "about" should be understood to encompass ± 5% of a specified amount or value.

The present disclosure includes compositions comprising at least one gelling agent, at least one salt, and water. The composition may be formulated as a biocompatible gel suitable for injection.

The at least one gelling agent may be natural (including natural gums such as vegetable gums and/or microbial gums) or synthetic in origin, and may be anionic, cationic, or neutral. Non-limiting examples of gelling agents suitable for the compositions herein include polysaccharides such as gellan gum, xanthan gum, gum arabic, guar gum, locust bean gum, alginate, and carrageenans.

In some embodiments the composition may comprise gellan gum, xanthan gum, or a mixture thereof. Gellan gum is a polysaccharide produced by *Sphingomonas* bacteria, and has a general structure formed of repeating units of four sugars linked together: two residues of D-glucose, one residue of L-rhamnose, and one residue of D-glucuronic acid. Xanthan gum is a polysaccharide produced by *Xanthomonas* bacteria, and has a general structure formed of repeating units of five sugars linked together: two residues of D-glucose, two residues of D-mannose, and one residue of D-glucuronic acid.

There are two types of gellan gum: native and deacylated. The structure of native gellan gum includes two acyl groups, acetate and glycerate, bound to the glucose residue adjacent to the glucuronic acid residue. These acyl groups may be removed under alkaline conditions to produce deacylated gellan gum, which results in different stability and plasticity properties in comparison to native gellan gum. For example, native gellan gum generally forms softer, more elastic gels with thermoreversibility, whereas deacylated gellan gum generally forms harder, more inelastic gels with higher heat resistance. The compositions herein may comprise native gellan gum, deacylated gellan gum, or a mixture thereof. In at least one embodiment, the composition comprises deacylated gellan gum.

Certain microbial extracts may comprise endotoxins, e.g., lipopolysaccharides from the bacteria that become combined with the polysaccharide structure. In some embodiments, the gelling agent may be chosen to minimize or eliminate the introduction of endotoxins into the composition, or may be processed to reduce or eliminate the concentration of endotoxins prior to use in the compositions disclosed herein.

According to some aspects of the present disclosure, the composition may comprise a microbial-sourced polysaccharide, e.g., xanthan gum, that has been processed to reduce the amount of endotoxins present, such that the resulting composition is pharmaceutically-acceptable and in compliance with the applicable government regulatory standards. For example, the at least one gelling agent may have an endotoxin level of 20 endotoxin units (EU) or less, such as from 0 EU to 20 EU, from 0 EU to 10 EU, from 0 EU to 5 EU, from 1 EU to 20 EU, from 1 EU to 10 EU, or from 1 EU to 5 EU. Thus, for example, a composition may have an endotoxin level of 20 EU or less, such as from 0 EU to 20 EU, from 0 EU to 10 EU, from 0 EU to 5 EU, from 1 EU to 20 EU, from 1 EU to 10 EU, or from 1 EU to 5 EU. In use, the composition may be delivered to a target site of a patient via a suitable medical device (e.g., a syringe or a fluid reservoir coupled to an injection needle). Thus, for example, the medical device may have an endotoxin level of 20 EU or less, such as from 0 EU to 20 EU, from 0 EU to 10 EU, from 0 EU to 5 EU, from 1 EU to 20 EU, from 1 EU to 10 EU, or from 1 EU to 5 EU. Bacterial endotoxin levels may be measured, for example, using the Limulus Amebocyte Lysate (LAL) test.

The concentration of gelling agent(s) may range from about 0.01% to about 2.0% by weight with respect to the total weight of the composition, such as from about 0.02% to about 1.5%, from about 0.05% to about 0.5%, from about 0.10% to about 1.0%, from about 0.10% to about 0.30%, or from about 0.02% to about 0.25% by weight with respect to the total weight of the composition, e.g., about 0.10%, about 0.15%, or about 0.20% by weight with respect to the total weight of the composition. In at least one embodiment, the total concentration of gelling agent(s) in the composition ranges from about 0.05% to about 0.5% by weight with respect to the total weight of the composition.

The at least one gelling agent may be combined with one or more biocompatible, e.g., physiologically compatible, salts. Non-limiting examples of salts suitable for the compositions herein include salts comprising sodium, calcium, magnesium, and/or potassium cations. The salts may include, for example, chloride salts, phosphate salts, and/or sulfate salts, such as, e.g., sodium chloride (NaCl), potassium chloride (KCl), calcium chloride (CaCl₂), sodium dihydrogen phosphate (NaH₂PO₄), potassium hydrogen phosphate (K₂HPO₄), magnesium sulfate (MgSO₄), sodium gluconate (C₆H₁₁NaO₇), sodium acetate trihydrate (C₂H₉NaO₅·3H₂O), and magnesium chloride (MgCl₂). For example, the at least one gelling agent may comprise an anionic polysaccharide, and the salt(s) may provide a source of monovalent or divalent cations compatible with the polysaccharide.

In some embodiments, the composition may comprise a physiologically compatible saline solution, such as, e.g., a sodium chloride solution. For example, the composition may comprise a 0.9% wt. sodium chloride solution, e.g., providing sodium cations to assist in formation of the three-dimensional solid gel network. In some embodiments, the composition may be isotonic. For example, the saline solution may have an appropriate concentration of monovalent and/or divalent cations such that the composition is isotonic with tissue fluids and/or blood. Other physiologically-compatible solutions comprising suitable ionic concentrations may be used to provide for isotonicity.

The concentration of salt of the composition may range from about 0.1% to about 2.0% by weight with respect to the total weight of the composition, such as from about 0.25% to about 1.0%, from about 0.5% to about 1.5%, or from about 0.5% to about 1.0% by weight with respect to the total weight of the composition, e.g., about 0.25%, about 0.5%, about 0.75%, or about 1.0% by weight with respect to the total weight of the composition. Further, for example, the composition may comprise a salt solution having an osmolality ranging from about 240 mOsmol/kg to about 340 mOsmol/kg (e.g., 290 mOsmol/kg ± 50 mOsmol/kg), such as from about 250 mOsmol/kg to about 320 mOsmol/kg, or from about 280 mOsmol/kg to about 300 mOsmol/kg. The solution may be physiologically compatible, e.g., having electrolyte levels, osmolality, and pH suitable for injection into a patient. The pH of the solution may be adjusted using a suitable base such as sodium hydroxide to increase pH and/or a suitable acid such as hydrochloric acid, or may adjusted by other means or with other substances providing for a biocompatible composition.

The composition may comprise one or more other biocompatible compounds or agents. For example, the composition may comprise a biocompatible dye or coloring agent, such as brilliant blue (e.g., Brilliant Blue FCF, also known as FD&C Blue 1) indigo carmine (also known as FD&C Blue 2), indigo carmine lake, FD&C Blue 1 lake, and methylene blue (also known as methylthioninium chloride). For example, the composition may comprise a dye or colorant to allow for identification of the submucosal tissue plane upon injection into tissue, e.g., to determine the amount of tissue to be removed and/or assess the risk of perforation. Any other suitable types of biocompatible agents may be used, e.g., to adjust the pH and/or tonicity of the composition as appropriate for injection into tissue. For example, the composition may comprise one or more stabilizers and/or preservatives. According to some aspects, the composition may comprise an additive such as epinephrine to limit superficial bleeding. The composition may include one or more additives that improve visualization of diseased tissue or that have a therapeutic effect. For example, the additive may be pharmaceutically active, e.g., actively fighting cancerous cells.

As mentioned above, the composition may be formulated as a gel. For example, to prepare the composition, the gelling agent(s) may be combined with at least one pharmaceutically-acceptable salt (and optionally one or more other compounds or agents as discussed above) in an aqueous solution. The resulting mixture may be heated at a temperature ranging from about 70°C to about 130°C, such as from about 80°C to about 125°C, from about 90°C to about 115°C, or from about 95°C to about 105°C, e.g., a temperature of about 70°C, about 75°C, about 80°C, about 85°C, about 90°C, about 95°C, about 100°C, about 105°C, about 110°C, about 115°C, about 120°C, about 125°C, or about 130°C. In some examples, a minimum temperature from about 70°C to about 85°C may be used. In some examples, the mixture may be heated to boiling, e.g., a temperature ≥ 100°C.

The composition may be sterilized according to any suitable method, e.g., autoclaving, gamma irradiation, or via electron beam. In at least one embodiment, the composition may be heated at a temperature sufficient for sterilization, e.g., autoclaved at a temperature of about 121°C. The composition may be sterilized according to any suitable method, e.g., autoclaving, gamma irradiation, or via electron beam.

The mixture may be heated for an amount of time sufficient to hydrate the gelling agent and allow for formation of the three-dimensional gel network. With respect to polysaccharides like gellan gum, it is believed that the polysaccharide molecules may undergo a coil to double-helix transition with decreasing temperature, which may lead to gel formation, e.g., depending on the ionic strength and pH of the solution. For example, gellan gum coil molecules may form double helices with a reduction in temperature, and these helices may aggregate to form junction zones, resulting in gelation. In water, at low ionic strength and neutral pH, aggregation of the helices may be impeded by electrostatic repulsion between negatively charged carboxylic groups on the gellan molecules. The addition of a salt and/or the reduction in pH may decrease intermolecular repulsion between the helices, thereby enhancing junction zone formation, and consequently, the gel strength. The addition of salt therefore may facilitate physical cross-linking in an aggregation-like process to form a continuous, three-dimensional gel network. This continuous, three-dimensional network may provide for a solid or quasi-solid gel capable of maintaining its three-dimensional form even when inverted while in an open container.

In some aspects of the present disclosure, the mixture of gelling agent(s) and salt solution may be heated for a time ranging from about 5 minutes to about 90 minutes, from about 10 minutes to about 60 minutes, from about 15 minutes to about 45 minutes, or from about 20 minutes to about 30 minutes, e.g., about 15 minutes, about 20 minutes, about 30 minutes, or about 45 minutes. The mixture may be heated with constant or intermittent stirring, e.g., with a magnetic stirrer or other appropriate mixing equipment. While heated the composition may form a low viscosity fluid. The heat then may be removed and the composition allowed to cool. For example, the composition may be cooled to a temperature ≤ 55°C or about 50°C. As the composition cools, it may increase in viscosity and set into a gel.

In some embodiments, the composition is allowed to cool without stirring or other agitation. In such cases, the composition may form a substantially homogeneous gel, e.g., a continuous solid. Thus, for example, the composition may have a substantially continuous, three-dimensional, solid or quasi-solid gel network, as opposed to an agglomerate of gel particles or a colloid mixture. In some embodiments, the composition may be agitated as it cools, e.g., by constant or intermittent stirring. In such cases, the agitation may at least partially disrupt the structure of the gel, e.g., breaking apart the three-dimensional network to form individual gel particles or gel fragments. Additionally or alternatively, the structure of the gel may be at least partially disrupted after the composition cools, e.g., by stirring, shaking, and/or transferring the composition between containers.

Without intending to be bound by theory, it is believed that the application of various forces (e.g., shear force, compression force, stress, friction, etc.) may affect the continuity of the three-dimensional gel network, which in turn may impact its properties prior to use in medical procedures such as tissue resection. For example, transferring the composition between containers prior to injection may lead to shearing of the three-dimensional structure of the gel when ultimately injected into a patient. In some cases, this may limit the effectiveness of the composition, e.g., by limiting the ability of the gel to separate tissue layers and/or reducing the amount of time the gel remains within the tissue (e.g., within submucosal tissue) prior to diffusion or absorption into the tissue. Preparation of the compositions according to the methods herein may minimize shearing of the continuous, three-dimensional network of the gel prior to injection. Thus, the gel may maintain its three-dimensional structure until the gel is injected through a needle, whereupon the structure may form fragments of the original continuous, three-dimensional network. Those gel fragments may have a diameter corresponding to the diameter of the injection needle, such that the fragments are as large as possible in-vivo to retain as much of the three-dimensional structure of the gel as possible. Injection of these larger-sized particles or fragments is believed to increase the amount of time the gel remains within the tissue.

Embodiments of the present disclosure therefore may prevent or minimize disruption of the continuous, solid gel structure prior to injection. For example, the composition may be prepared such that it sets into a continuous, three-dimensional gel network while the composition is inside a reservoir of a medical device, such as an injection device. The composition may form a substantially homogeneous gel solid or quasi-solid in the reservoir without the need to disrupt the gel structure by transferring between storage containers. Thus, shear and/or other forces to the gel composition may be minimized prior to injecting the gel into a patient. According to some aspects, the composition is not transferred from the reservoir into any other container prior to injection from the reservoir to a target site of a patient.

Reservoirs suitable for the compositions herein may include, for example, syringes (e.g., a syringe barrel compatible with a manual or automatic injection system), flexible pouches such as a plastic bag, and other fluid containers configured for use with a suitable injection needle. Exemplary materials suitable for the reservoir include, but are not limited to, cyclic olefin copolymer, cyclic olefin polymer, polypropylene, polycarbonate, polyvinyl chloride, and glass.

The reservoir may be directly coupled to a needle, e.g., via a Luer adapter or other suitable connection, or may be indirectly coupled to a needle via a flexible tube, such as a catheter. Non-limiting examples of needles coupled a reservoir via a flexible tube include Interject™ sclerotherapy needles by Boston Scientific. The needle may be a hypodermic needle, and may range from a size of 7 gauge (4.57 mm outer diameter (OD), 3.81 mm inner diameter (ID)) to 33 gauge (0.18 mm OD, 0.08 mm ID), e.g., a size of 16 gauge (1.65 mm OD, 1.19 mm ID), 21 gauge (0.82 mm OD, 0.51 mm ID), 22 gauge (0.72 mm OD, 0.41 mm ID), 23 gauge (0.64 mm OD, 0.33 ID), or 24 gauge (0.57 mm OD, 0.31 mm ID). Exemplary materials for the needle include, but are not limited to, metals and metal alloys, such as stainless steel and Nitinol, and polymers. The distal tip of the needle may be sharpened, and may have a beveled shape. The proximal end of the needle may include a suitable fitting/adaptor (e.g., a Luer adapter) for engagement with a syringe or other reservoir. In some embodiments, the needle may include an elongated tube or catheter between the needle tip and the proximal fitting/adapter.

As discussed above, the composition may be introduced into a suitable reservoir of a medical device (e.g., an injection device or an injection system) after heating the composition. For example, the mixture of gelling agent(s), salt(s), and water may be introduced into the reservoir after heating the mixture at a temperature ranging from about 70°C to about 130°C, such as a temperature ranging from about 90°C to about 110°C.

The composition subsequently may be allowed to cool and increase in viscosity to set into a homogeneous, solid or quasi-solid gel while inside the reservoir. In some embodiments, the composition may be re-heated after its introduction into the reservoir and subsequently allowed to cool to set into its final solid or quasi-solid, three-dimensional gel form. For example, the composition may undergo one or more heating/cooling cycles once introduced into the reservoir. According to some aspects, for example, the composition may be heated twice by initially heating the mixture of gelling agent(s), salt(s), and water (e.g., to ensure hydration), and then subsequently heating the composition after introducing the composition into the reservoir from which it will be injected, allowing it to cool and set into a gel with a continuous, three-dimensional structure. According to some aspects, the composition may not be transferred from the reservoir to any other container prior to injection from the reservoir directly to the target site of a patient.

The composition may be sterilized. For example, the composition may be autoclaved while inside the reservoir by heating the composition at or to a temperature of about 121°C, and the composition subsequently be allowed to cool and set into a homogeneous solid or quasi-solid gel inside the reservoir. Additionally or alternatively, the composition may be sterilized via gamma irradiation or by electron beam after its introduction into the reservoir.

In some aspects, the composition may be heated as discussed above and cooled in an initial storage container, such as a vial, and subsequently transferred into a suitable reservoir from which the composition may be injected into a patient. In such cases, the composition may be agitated as it cools in the initial container to form an agglomeration of smaller gel particles or gel-like fluid. Additionally or alternatively, the composition may be agitated, sheared, extruded, or otherwise broken up after cooling and housed in the storage container. The agglomeration of gel particles or gel-like fluid may be subsequently mixed with additional liquid components (e.g., other viscous agents, including viscous forms of gellan gum) after the gel has set. The composition then may be transferred from the storage container to a suitable reservoir, heated, and subsequently allowed to cool to set into a homogeneous gel inside the reservoir. The composition then may be injected directly from the reservoir through a needle to the target site of a patient. According to some aspects, the gel may be subjected to minimal shear forces and/or other forces prior to injection into a patient. The viscosity of the composition while set into gel form within the reservoir, prior to injection, may depend on the properties of the gelling agent(s) and/or the concentration of the gelling agent(s) relative to other components of the composition.

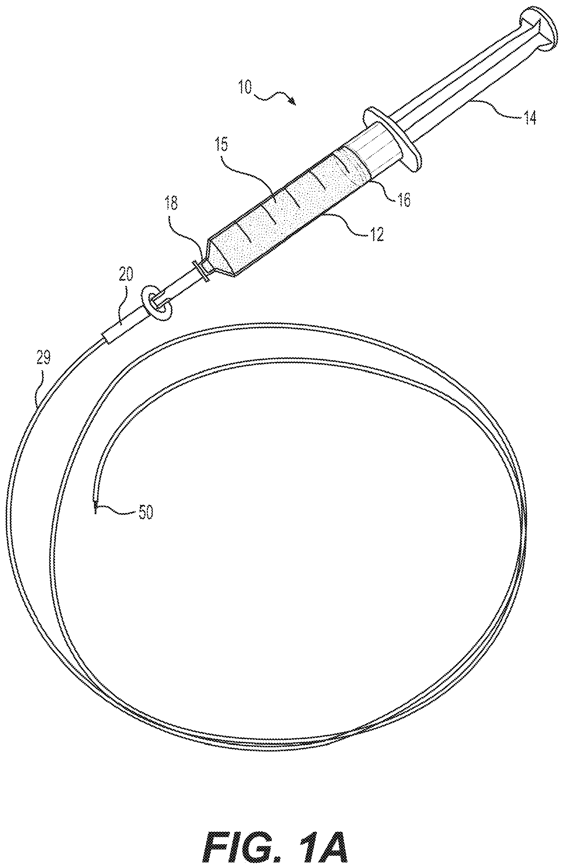

Fig. 1A illustrates an exemplary syringe 10 providing a reservoir for a gel composition as discussed above. The syringe 10 may comprise a barrel 12, a plunger 14, and one or more stoppers 16. The composition 15 may be prepared as discussed above and allowed to set into a solid gel with a continuous, three-dimensional structure across the diameter of the barrel 12. The barrel 12 may include a Luer adapter (or other suitable adapter/connector), e.g., at the distal end 18 of the barrel 12, for attachment to an injection needle 50 via a flexible catheter 29. The proximal end of the catheter 29 may include a suitable connection 20 for receiving the barrel 12. In other examples, the barrel 12 may be directly coupled to the injection needle 50. The syringe barrel 12 may serve as a reservoir, containing a gel composition 15 for injection through the needle 50.

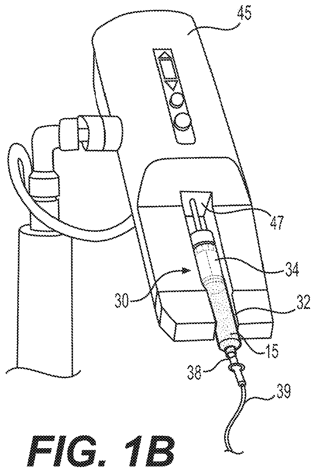

Fig. 1B illustrates an exemplary syringe 30 for use with an automatic injection system 45. The syringe 30 may include any of the features of the syringe 10 of Fig. 1A, e.g., a barrel 32, a plunger 34, and a Luer adapter (or other suitable adapter/connector) at the distal end 38 of the barrel 32. A composition 15 may be prepared as discussed above and allowed to set into a gel in the barrel 32, and the syringe 30 may be inserted into a channel 47 of the injection system 45 for automatic control over the amount of gel injected. The distal end 38 of the syringe 30 may be coupled to an injection needle (e.g., similar to injection needle 50 of Fig. 1A) via a catheter 39. According to some aspects of the present disclosure, the plunger 34 may form part of the injection system 45 and the barrel 32 may be a separate component, e.g., a replaceable cartridge, to be connected to the injection system 45. For example, the composition 15 may be prepared in the barrel 32 as a replaceable cartridge having a proximal attachment compatible with a plunger component of the injection system 45.

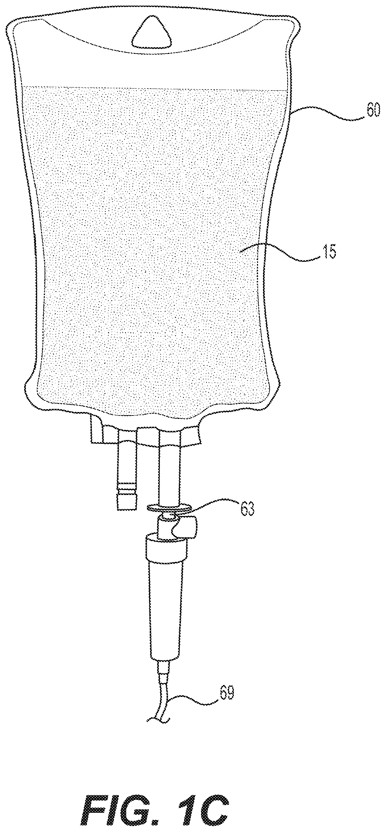

Fig. 1C illustrates an exemplary reservoir 60 according to some aspects of the present disclosure. The reservoir 60 may be provided by a flexible pouch or bag, such as an IV bag. A composition 15 may be prepared as discussed above and allowed to set into a gel in the reservoir 60. The reservoir 60 may be sterile, and may comprise a plastic material such as polyvinyl chloride (PVC) (e.g., with a plasticizer such as bis(2-ethylhexyl) phthalate (DEHP)) or a non-PVC plastic material. The pouch may include a Luer adapter 63 for attachment to a catheter 69 and/or needle (having any suitable gauge size, as described above) for injecting the composition 15 into a patient. The reservoir 60 may be compressible, e.g., to allow for delivery of the composition through the catheter 69 and/or needle by compression of the reservoir 60.

Reservoirs and injection methods other than those illustrated in Figs. 1A-1C may be used in according with the present disclosure. For example, the composition may be housed in a reservoir coupled to a fluid channel and/or needle that forms part of an electrocautery device or system. Thus, a physician may inject the composition through the fluid channel while simultaneously or subsequently operating other portions of the device or system, such as an electrocautery knife or snare.

The amount of force required to move the composition through a needle aperture (generally described as "peak load" force) may depend on the viscosity of the composition, the dimensions of the needle (inner diameter, outer diameter, and/or length), and/or the material(s) from which the needle is formed. For example, a greater amount of force may be applied to inject the composition through a 33 gauge needle in comparison to a 7 gauge needle. Additional factors that may affect the amount of force applied to inject the composition may include the dimensions of a catheter (inner diameter, outer diameter, and/or length) connecting the reservoir to the needle. Suitable peak loads for injection with one or two hands may range from about 5 lbf to about 25 lbf, such as from about 10 lbf to about 20 lbf, e.g., about 15 lbf. The loads measured for a given gel concentration may vary for different needles and flow rates.

According to some aspects of the present disclosure, the size of the needle may be chosen based on the viscosity and/or components of the composition, or vice versa. Further, the dimensions of the catheter tubing (inner diameter, outer diameter, and/or length), if any, may affect the types and amount of force applied to the composition during injection. These parameters may be taken into consideration according to the properties of the composition and the needs of the patient. According to some aspects of the present disclosure, the size of the needle may be 23 gauge or 25 gauge. In some cases, a larger size of 20 gauge, 21 gauge, or 22 gauge may be used to inject the compositions herein.

In some examples, the composition may be pseudoplastic. Pseudoplasticity generally refers to the property of decreasing in viscosity upon the application of shear force. Thus, for example, the composition may have a higher viscosity at rest or under low shear conditions (e.g., while stored in a container) than while under high shear conditions (e.g., during loading into and/or injection through a needle). Examples of materials that may exhibit pseudoplasticity include gellan gum and xanthan gum, among other types of polysaccharides.

The compositions herein may be used in various medical procedures, including tissue resection procedures of the GI system, the respiratory system, and/or the genitourinary system. The tissue resected in such medical procedures may comprise diseased or injured tissue, non-diseased tissue, or a combination thereof. Exemplary tissue resection procedures include endoscopic mucosal resection (EMR) and endoscopic submucosal dissection (ESD). In these procedures, an endoscope is typically inserted into the patient's esophagus and advanced through the GI system to reach the target site in the esophagus, stomach, or intestine. EMR is typically used for removal of tissue smaller than 2 cm in diameter, e.g., to biopsy tissue or to remove injured or diseased tissue (e.g., a cancerous lesion), while ESD is typically used for removal of larger lesions.

In some aspects, a continuous solid or quasi-solid gel composition may be prepared as discussed above and injected between two layers of tissue, e.g., injected into submucosal tissue between an upper mucosal layer and lower muscularis propria layer at a target treatment site. The composition may be injected within the submucosal space (submucosal layer) under a portion of tissue, whereupon the injected gel may cause the mucosal tissue to separate from the muscularis propria layer, elevating the mucosal tissue layer. A suitable cutting device, e.g., an electrocautery cutting device such as a knife, snare, scissors, or forceps, may then be used to remove the portion of tissue. For removal of larger portions of tissue (e.g., via ESD), the composition may be injected under the portion of tissue, wherein the gel elevates the upper layer of tissue from the lower layer. The cutting device then may be used to make an incision around the portion of tissue and remove it. The composition may be injected in the submucosal layer to assist in removing additional portions of tissue.

In some aspects, the composition may maintain separation of the tissue layers throughout the entire resection procedure. A portion of the gel composition may be removed via the resection process. Following tissue resection, remaining portions of the gel composition may be flushed from the site with water or saline, or may naturally diffuse into the tissue.

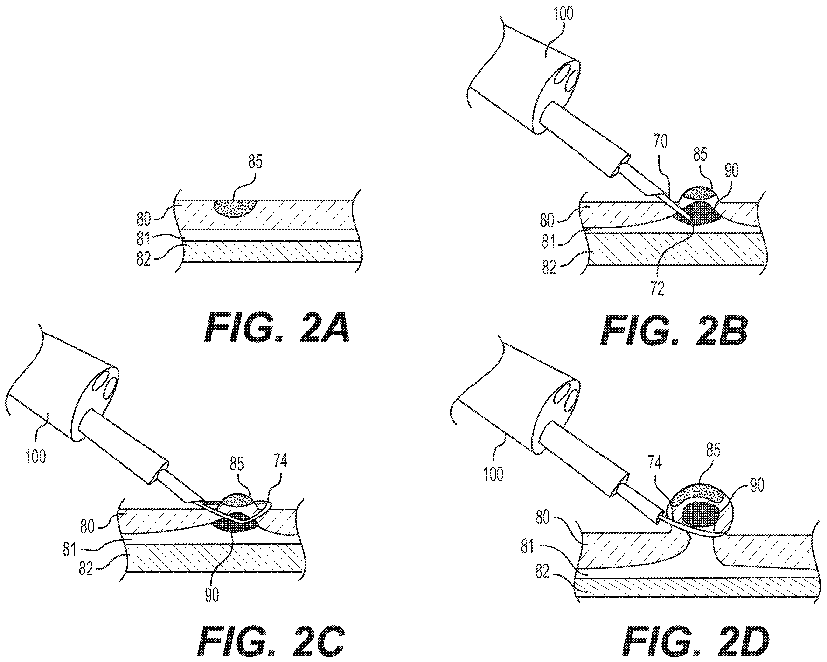

Figs. 2A-2E illustrate an exemplary resection procedure according to some aspects of the present disclosure. For example, the procedure may be EMR or ESD as discussed above, or any other suitable medical procedure for resecting tissue. Fig. 2A shows a cross-sectional view of two portions of tissue or tissue layers 80, 82, which may be separated by a middle layer 81 of tissue (such as, e.g., upper mucosal and lower muscularis propria layers separated by a middle submucosal tissue layer). One or both of the portions of tissue 80, 82 may include a section of tissue 85 targeted for removal. For example, the section of tissue 85 may comprise injured or diseased tissue, or may comprise tissue targeted for biopsy and subsequent analysis. In the example of Fig. 2A, the section of tissue 85 is located toward the tissue surface, however, the devices and compositions disclosed herein may be used to remove tissue from inner tissue layers.

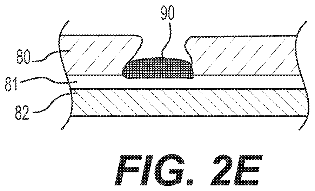

As shown in Fig. 2B, an endoscope 100 defining one or more lumens (e.g., three lumens as shown) may be used to deliver a needle 70 to the treatment site. The needle 70 may have a hollow lumen and a sharp, beveled tip 72 for piercing the tissue surface such that the needle tip 72 is within the middle layer 81 between the upper and lower portions of tissue 80, 82. The needle lumen may be in communication with a fluid reservoir, such as a syringe or other reservoir containing a continuous, solid gel composition 90 prepared as discussed above. The syringe may be used to inject the composition 90 into the middle layer 81 between the portions of tissue 80, 82 to form a cushion or bleb of gel, as shown in Fig. 2B. Once the composition 90 is injected, the volume of the gel 90 may cause the upper and lower portions of tissue 80, 82 to separate, such that the section of tissue 85 may be elevated from underlying tissue. An electrocautery snare 74 or other cutting device 74 (such as, e.g., an electrocautery knife, scissors, or forceps, among other suitable cutting devices) may be used to cut and remove the section of tissue 85, as shown in Figs. 2C and 2D. Once the section of tissue 85 is removed, as shown in Fig. 2E, a portion of the gel 90 may naturally diffuse into one or more of the tissue layers 80, 81, 82.

Other aspects and embodiments of the present disclosure will be apparent to those skilled in the art from consideration of the specification and practice of the embodiments disclosed herein. While certain features of the present disclosure are discussed within the context of exemplary tissue resection procedures, the compositions, systems, and methods may be used for other medical procedures according to the general principles disclosed.

### EXAMPLES

The following examples are intended to illustrate the present disclosure without, however, being limiting in nature. It is understood that the present disclosure encompasses additional aspects and embodiments consistent with the foregoing description and following examples.

### Example 1

Compositions A-C were prepared according to Table 1 below as follows. Gellan gum (Gelzan™ CM, CP Kelco) and erioglaucine disodium salt (FD&C Blue 1, Sigma Aldrich) were added to phosphate buffered saline (PBS) solution and continuously stirred with a magnetic stirrer and bar. The PBS solution was prepared by dissolving 1 package PBS powder (Sigma Aldrich) in 1000 ml deionized water to produce 0.138 M NaCl. The gellan gum/salt/PBS mixture was brought to a boil with stirring, turning from slightly cloudy blue to transparent blue in appearance. The solution was allowed to cool to room temperature (∼25°C) with stirring, resulting in a viscous fluid.

**Table 1**

| **Composition** | **Gellan gum (% w/w)** | **Erioglaucine disodium salt (% w/w)** | **PBS (% w/w)** |
|---|---|---|---|
| A | 0.10 | 0.004 | 99.90 |
| B | 0.15 | 0.004 | 99.85 |
| C | 0.20 | 0.004 | 99.80 |

Each solution was drawn up into a 10-ml syringe (BD Luer-Lok Tip). Each syringe was autoclaved at 121°C. The syringes were then allowed to cool to room temperature (∼25°C). The resulting syringe contents comprised a quasi-solid gel that maintained its shape upon inversion, but that could be injected through a needle.

A comparative composition (Composition D) was prepared with 0.20% xanthan gum (Sigma Aldrich), 0.004% erioglaucine disodium salt (FD&C Blue 1, Sigma Aldrich), and 99.8% phosphate buffered saline (PBS) solution, and continuously stirred with a magnetic stirrer and bar. The resulting solution formed a Composition D in the form of a viscous fluid, rather than a quasi-solid gel as for gellan gum Compositions A-C. The viscous fluid of the xanthan gum Composition D was capable of flowing upon inversion.

### Example 2

The syringes containing gellan gum Compositions A-C prepared according to Example 1 were connected to an injection needle (Boston Scientific Interject™ 23ga Needle) and injected at 3 mm/s (0.5 ml/s flow rate) using an Instron 5564 Universal Testing Machine and 500N load cell and custom fixtures. The measured peak load values were observed to vary based on gellan gum concentration, as shown in Table 2.

**Table 2**

| **Composition** | **Average Peak Load (lbf)** |
|---|---|
| A | 13.28 |
| B | 17.60 |
| C | 22.87 |

It is intended that the specification and examples be considered as exemplary only, with a true scope of the present disclosure being indicated by the following claims.

## Claims

1. A method of preparing a composition (15) for delivery to a target site of a patient, the method comprising:
combining gellan gum, at least one salt, and water to form a mixture;
heating the mixture at a temperature ranging from about 70°C to about 130°C;
introducing the mixture into a reservoir (12, 60); and
allowing the mixture to cool while inside the reservoir to form a gel having a continuous, three-dimensional structure inside the reservoir;
wherein the composition is biocompatible and injectable from the reservoir through a needle (50) to the target site.

2. The method of claim 1, wherein the composition comprises 0.01% to 2.0% gellan gum by weight with respect to the total weight of the composition.

3. The method of claim 1 or 2, wherein the composition comprises 0.05% to 0.5% gellan gum by weight with respect to the total weight of the composition.

4. The method of any of the preceding claims, wherein the mixture is introduced into the reservoir after heating the mixture at a temperature ranging from about 70°C to about 130°C.

5. The method of any of the preceding claims, wherein the temperature of the mixture when being introduced into the reservoir ranges from about 70°C to about 130°C.

6. The method of any of claims 1-4, further comprising allowing the mixture to cool before introducing the mixture into the reservoir, and heating the mixture at a temperature ranging from about 70°C to about 130°C while inside the reservoir.

7. The method of any of the preceding claims, wherein heating the mixture sterilizes the mixture.

8. The method of any of the preceding claims, wherein the reservoir is a barrel (12) of a syringe (10).

9. The method of any of claims 1-7, wherein the reservoir comprises a flexible pouch (60).

10. The method of any of the preceding claims, wherein the reservoir is coupled to the needle by a flexible tube (29).

11. The method of any of the preceding claims, wherein the composition comprises a coloring agent.

12. The method of any of the preceding claims, wherein the continuous, three-dimensional structure of the gel extends across an entire cross-sectional dimension of the reservoir.

13. A medical device comprising the composition prepared according to claim 1, wherein the composition comprises 0.05% to 0.5% gellan gum by weight with respect to the total weight of the composition.

14. The medical device of claim 13, wherein the medical device comprises the reservoir, the needle, and a flexible tube connecting the reservoir to the needle, and wherein the reservoir is a barrel of a syringe or a flexible pouch.

15. The medical device of claim 13, wherein the medical device has an endotoxin level of 20 endotoxin units (EU) or less.

## Patentansprüche

1. Verfahren zum Herstellen einer Zusammensetzung (15) zur Abgabe an eine Zielstelle eines Patienten, wobei das Verfahren die Schritte aufweist:
Kombinieren von Gellangummi, mindestens eines Salzes und Wasser zum Herstellen eines Gemischs;
Erwärmen des Gemischs bei einer Temperatur im Bereich von etwa 70°C bis etwa 130°C;
Einbringen des Gemischs in ein Reservoir (12, 60); und
Abkühlenlassen des Gemischs, während es sich im Reservoir befindet, um ein Gel mit einer kontinuierlichen, dreidimensionalen Struktur im Reservoir zu bilden,
wobei die Zusammensetzung biokompatibel ist und aus dem Reservoir durch eine Nadel (50) an die Zielstelle injizierbar ist.

2. Verfahren nach Anspruch 1, wobei die Zusammensetzung, bezogen auf das Gesamtgewicht der Zusammensetzung, 0,01 Gew.-% bis 2,0 Gew.-% Gellangummi aufweist.

3. Verfahren nach Anspruch 1 oder 2, wobei die Zusammensetzung, bezogen auf das Gesamtgewicht der Zusammensetzung, 0,05 Gew.-% bis 0,5 Gew.-% Gellangummi aufweist.

4. Verfahren nach einem der vorangehenden Ansprüche, wobei die Mischung in das Reservoir eingebracht wird, nachdem die Mischung bei einer Temperatur im Bereich von etwa 70°C bis etwa 130°C erwärmt wurde.

5. Verfahren nach einem der vorangehenden Ansprüche, wobei die Temperatur des Gemischs beim Einbringen in das Reservoir im Bereich von etwa 70°C bis etwa 130°C liegt.

6. Verfahren nach einem der Ansprüche 1 bis 4, ferner mit dem Abkühlenlassen der Mischung vor dem Einbringen der Mischung in das Reservoir, und dem Erwärmen der Mischung bei einer Temperatur im Bereich von etwa 70°C bis etwa 130°C, während sie sich im Reservoir befindet.

7. Verfahren nach einem der vorangehenden Ansprüche, wobei das Erwärmen des Gemischs das Gemisch sterilisiert.

8. Verfahren nach einem der vorangehenden Ansprüche, wobei das Reservoir ein Zylinder (12) einer Spritze (10) ist.

9. Verfahren nach einem der Ansprüche 1 bis 7, wobei das Reservoir einen flexiblen Beutel (60) aufweist.

10. Verfahren nach einem der vorangehenden Ansprüche, wobei das Reservoir über einen flexiblen Schlauch (29) mit der Nadel verbunden ist.

11. Verfahren nach einem der vorangehenden Ansprüche, wobei die Zusammensetzung ein Färbemittel aufweist.

12. Verfahren nach einem der vorangehenden Ansprüche, wobei sich die kontinuierliche, dreidimensionale Struktur des Gels über eine gesamte Querschnittsabmessung des Reservoirs erstreckt.

13. Medizinische Vorrichtung mit der nach Anspruch 1 hergestellten Zusammensetzung, wobei die Zusammensetzung, bezogen auf das Gesamtgewicht der Zusammensetzung, 0,05 Gew.-% bis 0,5 Gew.-% Gellangummi aufweist.

14. Medizinische Vorrichtung nach Anspruch 13, wobei die medizinische Vorrichtung das Reservoir, die Nadel und einen flexiblen Schlauch aufweist, der das Reservoir mit der Nadel verbindet, und wobei das Reservoir ein Zylinder einer Spritze oder ein flexibler Beutel ist.

15. Medizinische Vorrichtung nach Anspruch 13, wobei die medizinische Vorrichtung einen Endotoxinwert von 20 Endotoxineinheiten (EU) oder weniger aufweist.

## Revendications

1. Procédé de préparation d'une composition (15) pour libération sur un site cible d'un patient, le procédé comprenant :
la combinaison de gomme gellane, d'au moins un sel, et d'eau pour former un mélange ;
le chauffage du mélange à une température d'environ 70°C à environ 130°C ;
l'introduction du mélange dans un réservoir (12, 60) ; et
le laisser refroidir le mélange alors qu'il est à l'intérieur du réservoir pour former un gel ayant une structure tridimensionnelle, continue à l'intérieur du réservoir ;
dans lequel la composition est biocompatible et injectable à partir du réservoir par l'intermédiaire d'une aiguille (50) au site cible.

2. Procédé selon la revendication 1, dans lequel la composition comprend de 0,01 % à 2,0 % de gomme gellane en masse par rapport à la masse totale de la composition.

3. Procédé selon la revendication 1 ou 2, dans lequel la composition comprend de 0,05 % à 0,5 % de gomme gellane en masse par rapport à la masse totale de la composition.

4. Procédé selon l'un quelconque des revendications précédentes, dans lequel le mélange est introduit dans le réservoir après chauffage du mélange à une température d'environ 70°C à environ 130°C.

5. Procédé selon l'une quelconque des revendications précédentes, dans lequel la température du mélange lorsqu'il est introduit dans le réservoir est d'environ 70°C à environ 130°C.

6. Procédé selon l'une quelconque des revendications 1-4, comprenant de plus le laisser refroidir le mélange avant l'introduction du mélange dans le réservoir, et le chauffage du mélange à une température d'environ 70°C à environ 130°C tout en étant à l'intérieur du réservoir.

7. Procédé selon l'une quelconque des revendications précédentes, dans lequel le chauffage du mélange stérilise le mélange.

8. Procédé selon l'une quelconque des revendications précédentes, dans lequel le réservoir est un cylindre (12) d'une seringue (10).

9. Procédé selon l'une quelconque des revendications 1-7, dans lequel le réservoir comprend une poche flexible (60).

10. Procédé selon l'une quelconque des revendications précédentes, dans lequel le réservoir est couplé à l'aiguille par un tube flexible (29).

11. Procédé selon l'une quelconque des revendications précédentes, dans lequel la composition comprend un agent colorant.

12. Procédé selon l'une quelconque des revendications précédentes, dans lequel la structure tridimensionnelle, continue du gel s'étend à travers une dimension transversale entière du réservoir.

13. Dispositif médical comprenant la composition préparée selon la revendication 1, dans lequel la composition comprend de 0,05 % à 0,5 % de gomme gellane en masse par rapport à la masse totale de la composition.

14. Dispositif médical selon la revendication 13, dans lequel le dispositif médical comprend le réservoir, l'aiguille, et un tube flexible connectant le réservoir à l'aiguille, et dans lequel le réservoir est un cylindre d'une seringue ou une poche flexible.

15. Dispositif médical selon la revendication 13, dans lequel le dispositif médical présente une teneur en endotoxine de 20 unités d'endotoxine (EU) ou inférieure.
